Europäisches Patentamt

European Patent Office

Office européen des brevets

(19)

(11) Publication number: **0 069 351**
**B1**

(12) # EUROPEAN PATENT SPECIFICATION

(45) Date of publication of patent specification: 05.11.86

(51) Int. Cl.⁴: **A 61 B 17/36**

(21) Application number: **82105881.5**

(22) Date of filing: **01.07.82**

(54) **Hand piece for use with surgical laser knife device.**

(30) Priority: **02.07.81 JP 102230/81**
**13.07.81 JP 108108/81**

(43) Date of publication of application:
**12.01.83 Bulletin 83/02**

(45) Publication of the grant of the patent:
**05.11.86 Bulletin 86/45**

(84) Designated Contracting States:
**DE FR GB SE**

(56) References cited:
**EP-A-0 069 389**
**US-A-3 622 743**
**US-A-3 821 510**
**US-A-3 991 764**

(73) Proprietor: **SUMITOMO ELECTRIC INDUSTRIES LIMITED**
**No. 15, Kitahama 5-chome Higashi-ku**
**Osaka-shi Osaka 541 (JP)**

(72) Inventor: **Kazuhito, Murakami c/o Sumitomo Electric Ind. Ltd.**
**Osaka Works No. 1-3, Shimaya 1-chome Konohana-ku**
**Osaka-shi Osaka (JP)**
Inventor: **Kenichi, Takahashi c/o Sumitomo Electric Ind. Ltd.**
**Osaka Works No. 1-3, Shimaya 1-chome Konohana-ku**
**Osaka-shi Osaka (JP)**
Inventor: **Shinya, Takenaka c/o Sumitomo Electric Ind. Ltd.**
**Osaka Works No. 1-3, Shimaya 1-chome Konohana-ku**
**Osaka-shi Osaka (JP)**
Inventor: **Katsuyoshi, Sunato c/o Sumitomo Electric Ind. Ltd.**
**Osaka Works No. 1-3, Shimaya 1-chome Konohana-ku**
**Osaka-shi Osaka (JP)**

(74) Representative: **Henkel, Feiler, Hänzel & Partner**
**Möhlstrasse 37**
**D-8000 München 80 (DE)**

Courier Press, Leamington Spa, England.

## Description

The present invention relates to a handpiece for a laser knife device, comprising an optical fibre passing through a tubular holder and being secured to the proximal end of the holder, the fibre extending through the distal end of the holder into and terminating within a tube connected to said distal end.

In using a laser knife device, it is important to direct laser beam exactly to a point to be surgically operated by an operator. In order to achieve this requirement, a handpiece has been used.

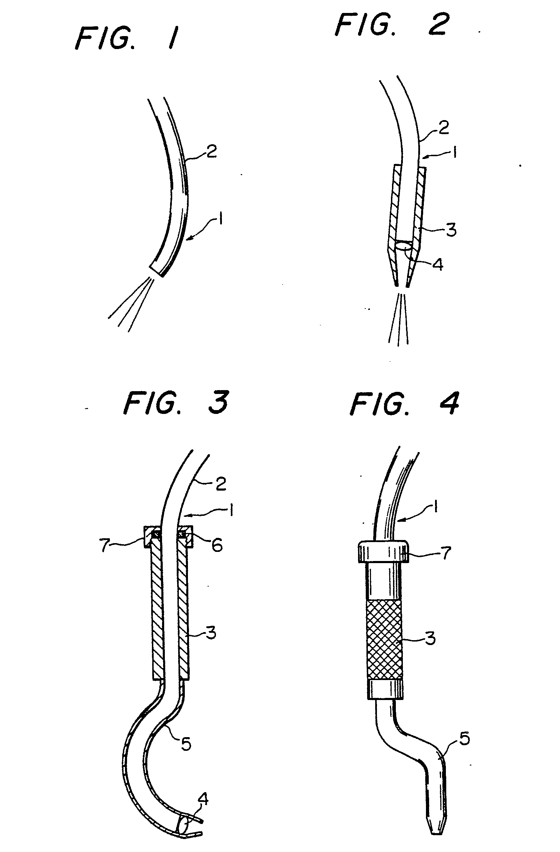

An example of the conventional handpiece for this purpose is shown in Fig. 1, in which the handpiece is a mere end portion of the optical fibre 1 having a coating 2. For the handpiece such as shown in Fig. 1, it is dangerous to hold the end portion of the optical fibre by hand and, even if the end portion is held by hand, it is very difficult to keep the direction of laser beam from the end of the optical fibre 1, which is relatively flexible, stably.

Another example of the conventional handpiece is shown in Fig. 2 in which the handpiece is composed of a nozzle type tube 3 fitted on the end portion of the optical fibre 1 and a lens 4 supported by an end portion of the tube 3 for converging laser beam. With this handpiece, the direction of laser beam is limited to an axis of the tube 3. Therefore, since the focus point is hidden by the handpiece itself it may be difficult to exactly irradiate a point in a limited space with laser beam or it may be difficult for the operator to direct laser beam to such point while directly looking at the latter point.

In US—A—3 821 510, a handpiece for use with a laser knife device as initially mentioned is disclosed. This known handpiece utilizes an optical fibre as an optical waveguide, comprising a flexible tube adapted to receive an end portion of the optical fibre so that an end of the optical fibre is held at around an end of said tube. A hand-manipulative instrument serves as a holder portion for the optical fibre and contains an adjustable laser-focusing system. However, this hand-manipulative instrument is rather complicated.

The handpiece of the initially mentioned kind is also described in EP—A—0 069 389, which handpiece is detachably coupled to the front end portion of the optical fibre and is so shaped in advance as to be able to confront the part to be irradiated with a laser beam. However, because this known handpiece is preformed in a given shape, the laser beam cannot be directed exactly to any desired point. This patent falls within the terms of Art. 54 (3) EPC.

An object of the present invention is to provide a handpiece for a laser knife device of the initially indicated kind by which it is possible to direct a laser beam exactly to a point under any space conditions.

The above object is achieved, according to the present invention, by a handpiece which is characterized in that the tube is either flexible, or is pivotable about an axis transverse the axis of the holder.

## Brief Description of the Drawing

Figs. 1 and 2 are cross sections of the conventional handpieces for laser knife device, respectively;

Fig. 3 is a cross section of one embodiment of the present invention;

Figs. 4 and 5 are modifications of configuration of the irradiating portion, respectively;

Fig. 6 is a cross section of the holder portion of the handpiece, showing a cooling passage formed in a holder portion of the handpiece for cooling the latter heated due to heat generated upon laser beam emission through the optical fibre; and

Fig. 7 is a side view of another embodiment of the present invention.

## Detailed Description of the Preferred Embodiments

In Fig. 3 which shows one embodiment of the present invention in cross section, a handpiece is composed of a holder 3 formed with a through-hole whose diameter is slightly larger than that of the optical fibre 1 so that the latter can be inserted there through and an irradiating portion in the form of a bent tube 5. An inner diameter of the tube 5 corresponds to that of the through-hole of the holder 3 and a rear end of the tube 5 is coaxially connected suitably to a front end of the holder 3 to form a long through-hole extending throughout the handpiece. A converging lens 4 is provided at a front end of the bent tube 5.

An optical fibre 1 is inserted from a rear end opening of the through-hole of the holder 3 into the handpiece with an end of the optical fibre 1 facing to the lens 4 by which laser beam from the end of the optical fibre is converged. An outer surface of the rear end of the holder 3 is threaded and a cap member 7 is fitted through an O ring 6 on the thread of the rear end of the holder 3 to support the optical fibre disposed in the handpiece tightly.

In this case the coating 2 of the optical fibre 1 in at least the bent tube 5 may be removed if necessary.

With the use of the detachable cap member 7 as means for fixing a positional relation between the handpiece and the optical fibre, it is possible to change the handpiece from one configuration to another dependant on specific application thereof.

With the handpiece thus constructed, laser beam is supplied from a $CO_2$ laser, guided by the optical fibre and emitted from the front end opening of the bent tube 5. Since the bent tube 5 is relatively small in diameter, it does not make an obstacle to the operation sight. Furthermore, since the bent tube 5 is relatively large in length, the distance between the operator's hand holding the holder portion 3 and the laser beam emitting point is large, causing the operator's hand to be safe.

Particularly, in the embodiment shown in Fig. 3 the bending of the tube 5 is substantial and takes in the form of semicircle, the axial direction of the holder portion 3 makes a substantial right angle to the direction of laser beam and thus it is possible to direct laser beam exactly to a point in a limited and irregularly shaped space. Therefore the handpiece of this embodiment is preferably applied to the fields of dental and/or stomatological surgery which is to be performed in a limited space.

The configuration of the bent tube 5 may be arbitrarily selected according to specific application thereof. An example of the bent tube configuration is shown in Fig. 4 in which a front portion of the bent tube 5 is made in parallel to the axis of the holder 3 with a small distance therebetween. Therefore, the operation point may be observed clearly by the operator, making this handpiece having the parallel bent tube 5 possible to use in the micro surgical operation.

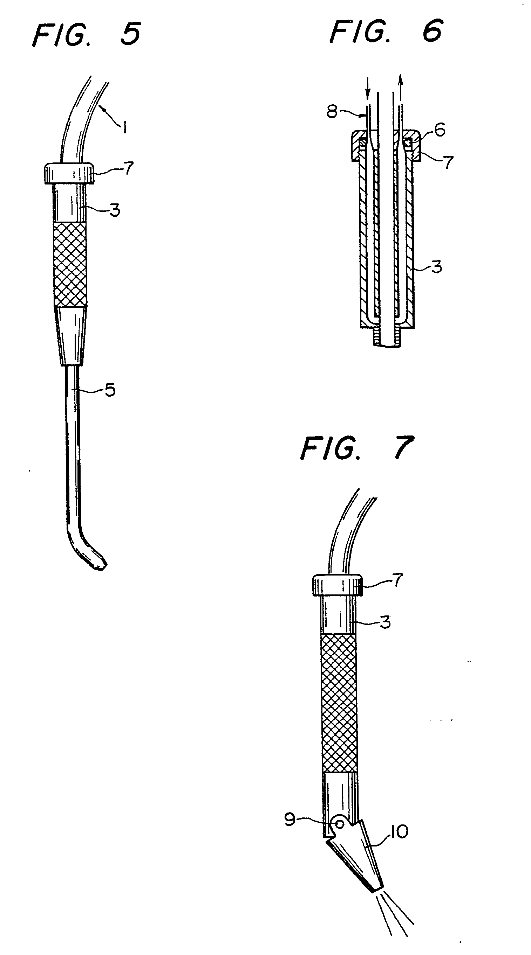

Another example shown in Fig. 5 is the bent tube 5 having a long and straight rear portion and a short front portion bent. This configuration of the bent tube 5 may be suitable to use in an operation to a deep portion, such as otorhinolaryngologic operation.

It has been known that the optical fibre 1 is heated by laser energy and thus the holder 3 is heated thereby. Therefore, it is desired to cool at least the holder 3 so that the operator can continue an operation.

Fig. 6 shows another embodiment by which at least the holder 3 is prevented from being heated by the heated optical fibre 1.

In Fig. 6, a cooling system for the holder portion 3 is provided. In this embodiment a fluid passage 8 is formed therein. The fluid passage is composed of an incoming passage portion formed in one side of and extending along the holder portion, a return passage portion formed in the other side of and extending along the holder portion and a connecting portion connecting ends of the passages at the end portion of the holder portion 3. A cooling medium is supplied to the incoming passage portion and flows through the connecting portion and the return passage portion.

It may be possible to color the end portion of the bent portion 5 black to prevent a possible reflection of laser beam.

Fig. 7 shows a further embodiment in which, instead of the bent tube, a movable pipe 10 is used. The movable pipe 10 is pivotally connected to the end of the holder portion 3 by a pin 9. The movable pipe 10 is pivoted around the pin 9 by positively applying a force to change the irradiating direction of laser beam.

With this structure, it is possible to flow an assist gas such as air through small gap formed between the outer surface of the optical fibre 2 and an inner wall of the movable pipe 10. The cooling medium passage such as shown in Fig. 6 may also be formed in the holder portion 3 of this embodiment.

As mentioned hereinbefore, according to the present invention, the handpiece for the laser knife device is composed of the holder portion and the semiflexible tube or movable pipe. Therefore, it is easy to change the laser beam direction by merely bending the semiflexible tube or merely pivotting the movable pipe. Therefore, the surgical operation can be performed exactly and safely. Furthermore, the holder portion may be cooled and there is no possibility of leakage of laser beam outside the handpiece even if the portion of the optical fibre within the semiflexible tube or the movable pipe is damaged, because laser light leaked from the damaged fibre may be reflected by the inner wall of the tube or pipe.

**Claims**

1. A handpiece for a laser knife device, comprising an optical fibre (1) passing through a tubular holder (3) and being secured to the proximal end of the holder, the fibre extending through the distal end of the holder into and terminating within a tube (5, 10) connected to said distal end, characterized in that the tube is either flexible, or is pivotable about an axis transverse to the axis of the holder.

2. A handpiece as claimed in claim 1, wherein said holder (3) is formed therein with a cooling medium passage (8) for cooling said holder (3).

3. A handpiece as claimed in claim 1 or 2, wherein the laser is a $CO_2$ laser.

4. A handpiece as claimed in claim 1, 2 or 3, wherein said holder (3) has the proximal end threaded and includes a cap (7) adapted to be screwed through an O ring (6) onto said the distal end to thereby fix the optical fibre (1) therein.

**Patentansprüche**

1. Handgriff für ein Lasermesser, umfassend einen Lichtleitfaserstrang (1), der einen rohrförmigen Halter (3) durchsetzt und am proximalen Ende des Halters befestigt ist, wobei sich der Lichtleitfaserstrang durch das distale Ende des Halters in ein mit diesem distalen Ende verbundenes Rohr (5, 10) erstreckt und in diesem endet, dadurch gekennzeichnet, daß das Rohr entweder biegsam oder um eine quer zur Achse des Halters verlaufende Achse schwenkbar ist.

2. Handgriff nach Anspruch 1, dadurch gekennzeichnet, daß im Halter (3) ein Kühlmitteldurchgang (8) zum Kühlen des Halters (3) ausgebildet ist.

3. Handgriff nach Anspruch 1 oder 2, dadurch gekennzeichnet, daß der Laser ein $CO_2$-Laser ist.

4. Handgriff nach Anspruch 1, 2 oder 3, dadurch gekennzeichnet, daß der Halter (3) am proximalen Ende ein Gewinde aufweist und mit einer Kappe (7) versehen ist, die unter Zwischenfügung eines O-Rings (6) auf das distale Ende aufschraubbar ist, um damit den Lichtleitfaserstrang (1) darin (im Halter) festzulegen.

**Revendications**

1. Pièce à main destinée à un scalpel à laser, comprenant une fibre optique (1) passant dans un support tubulaire (3) et fixée à l'extrémité interne du support, la fibre rejoignant l'extrémité externe du support dans un tube (5, 10) raccordé à ladite extrémité externe et dans lequel elle se termine, caractérisée en ce que le tube est soit souple, soit pivotant autour d'un axe transversal à l'axe du support.

2. Pièce à main selon la revendication 1, dans laquelle le support (3) a un passage (8) de circulation d'un fluide de refroidissement du support (3).

3. Pièce à main selon l'une des revendications 1 et 2, dans laquelle le laser est un laser à $CO_2$.

4. Pièce à main selon l'une quelconque des revendications 1, 2 et 3, dans laquelle le support (3) a son extrémité interne filetée, et comporte un capuchon (7) destiné à être vissé sur l'extrémité externe avec interposition d'un joint torique (6) afin que la fibre optique (1) soit fixée à l'intérieur.

0 069 351

FIG. 1

FIG. 2

FIG. 3

FIG. 4

1

0 069 351

FIG. 5

FIG. 6

FIG. 7

. 2